# EUROPEAN PATENT APPLICATION

(11) **EP 2 277 515 A1**
(43) Date of publication of application: **26.01.2011**
(21) Application number: 09305669.5
(22) Date of filing: 10.07.2009
(51) Int. Cl.: A61K 31/165, A61P 25/00

(54) **Use of the combination of teriflunomide and interferon beta for treating multiple sclerosis**

(71) Applicant: Sanofi-Aventis, 75013 Paris (FR)
(72) Inventor: Byrnes, William, Bridgewater, NJ 08807 (US); Douillet, Patrick, 75013, Paris (FR); Frangin, Gerald, 75013, Paris (FR)
(74) Representative: Romanowski, Caroline

(57) **Abstract**

This invention is related to the use of the combination of teriflunomide or a pharmaceutically acceptable salt thereof and interferon beta-1a or 1 b for treating multiple sclerosis.

## Description

### Field of the Invention

This invention is related to the use of the combination of teriflunomide or a pharmaceutically acceptable salt thereof and interferon beta for treating multiple sclerosis.

### Background of the Invention

Multiple sclerosis (MS) is a debilitating, inflammatory, neurological illness characterized by demyelination of the central nervous system. The disease primarily affects young adults with a higher incidence in females. Symptoms of the disease include fatigue, numbness, tremor, tingling, dysesthesias, visual disturbances, dizziness, cognitive impairment, urologic dysfunction, decreased mobility, and depression. Four types classify the clinical patterns of the disease: relapsing-remitting, secondary progressive, primary-progressive and progressive-relapsing (S.L. Hauser and D.E. Goodkin, Multiple Sclerosis and Other Demyelinating Diseases in Harrison's Principles of Internal Medicine 14th Edition, vol. 2, Mc Graw-Hill, 1998, pp. 2409-2419).

The exact etiology of MS is unknown; however, it is strongly suspected that the demyelination characteristic of the disease is the result of an autoimmune response perhaps triggered by an environmental insult, e.g. a viral infection. Specifically, it is hypothesized that MS is caused by a T-cell-mediated, autoimmune inflammatory reaction. The autoimmune basis is strongly supported by the fact that antibodies specific to myelin basic protein (MBP) have been found in the serum and cerebrospinal fluid of MS patients and these antibodies along with T-cells that are reactive to MBP and other myelin proteolipids increase with disease activity. Furthermore, at the cellular level it is speculated that T-cell proliferation and other cellular events, such as activation of B cells and macrophages and secretion of cytokines accompanied by a breakdown of the blood-brain barrier can cause destruction of myelin and oligodendrocytes. (R.A. Adams, M.V. Victor and A.H. Ropper eds, Principles of Neurology, Mc Graw-Hill, New York, 1997, pp.903-921). Progressive MS (primary and secondary) may be based on a neurodegenerative process occurring with demyelination.

The use of (Z)-2-cyano-3-hydroxy-but-2-enoic acid-(4'-trifluoromethylphenyl)-amide (also known as teriflunomide, Formula I) for treating multiple sclerosis has been disclosed in U.S. Patent No. 6,794,410. It is also disclosed therein that teriflunomide could possibly combine with another compound known to be effective for treating multiple sclerosis to treat the disease. However, no clinical result has been reported to prove the efficacy and safety of the combination.

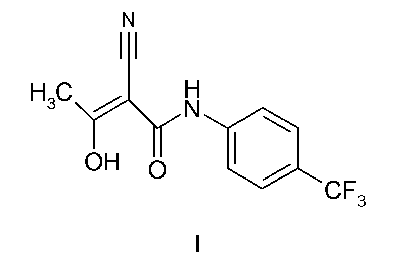

### Summary of the Invention

This invention is related to a method for treating multiple sclerosis, in a patient in need thereof, comprising administering to the patient about 7 mg or 14 mg teriflunomide or a pharmaceutically acceptable salt thereof, and a pharmaceutically effective amount of interferon beta-1a or 1b.

### Detailed Description of the Invention

As used above, and throughout the description of the invention, the following terms, unless otherwise indicated, shall be understood to have the following meanings:
"Patient" means a warm blooded animal, such as for example rat, mice, dogs, cats, guinea pigs, and primates such as humans.
"Pharmaceutically acceptable salts" means either an acid addition salt or a basic addition salt which is compatible with the treatment of patients for the intended use.
"Pharmaceutically acceptable acid addition salt" is any non-toxic organic or inorganic acid addition salt of the base compounds. Illustrative inorganic acids which form suitable salts include hydrochloric, hydrobromic, sulfuric and phosphoric acid and acid metal salts such as sodium monohydrogen orthophosphate and potassium hydrogen sulfate. Illustrative organic acids which form suitable salts include the mono-, di- and tri-carboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicylic, 2-phenoxybenzoic, p-toluenesulfonic acid and sulfonic acids such as methanesulfonic acid and 2-hydroxyethanesulfonic acid. Either the mono- or di-acid salts can be formed, and such salts can exist in either a hydrated, solvated or substantially anhydrous form. In general, the acid addition salts of these compounds are more soluble in water and various hydrophilic organic solvents and which in comparison to their free base forms, generally demonstrate higher melting points.
"Pharmaceutically acceptable basic addition salts" means non-toxic organic or inorganic basic addition salts of the compounds. Examples are alkali metal or alkaline-earth metal hydroxides such as sodium, potassium, calcium, magnesium or barium hydroxides; ammonia, and aliphatic, alicyclic, or aromatic organic amines such as methylamine, trimethylamine and picoline. The selection criteria for the appropriate salt is known to one skilled in the art.
"Pharmaceutically effective amount" means an amount of a compound/composition according to the present invention effective in producing the desired therapeutic effect.
"Stable dose of interferon beta-1a or 1b" means administering, for example, about 30 mcg beta-1a once a week, about 22 mcg beta-1a three times a week, about 44 mcg beta-1a three times a week, or 0.25 mg beta-1b every other day.
"Treat" or "treating" means to alleviate symptoms, eliminate the causation of the symptoms either on a temporary or permanent basis, or to prevent or slow the appearance of symptoms of the named disorder or condition.

One particular embodiment of the invention is a method for treating multiple sclerosis, in a patient in need thereof, comprising administering to the patient about 7 mg or 14 mg teriflunomide once a day, and a stable dose of interferon beta-1a or 1b.

Another particular embodiment of the invention is related to a clinically proven safe and effective method for treating multiple sclerosis, in a patient in need thereof, comprising administering to the patient about 7 mg or 14 mg teriflunomide once a day, and a stable dose of interferon beta-1a or 1b.

Another particular embodiment of the invention is related to a method for treating multiple sclerosis, in a patient in need thereof, comprising administering to the patient about 7 mg teriflunomide once a day and about 30 mcg beta-1a once a week.

Another particular embodiment of the invention is related to a method for treating multiple sclerosis, in a patient in need thereof, comprising administering to the patient about 7 mg teriflunomide once a day and about 22 mcg beta-1a three times a week.

Another particular embodiment of the invention is related to a method for treating multiple sclerosis, in a patient in need thereof, comprising administering to the patient about 7 mg teriflunomide once a day and about 44 mcg beta-1a three times a week.

Another particular embodiment of the invention is related to a method for treating multiple sclerosis, in a patient in need thereof, comprising administering to the patient about 7 mg teriflunomide once a day and about 0.25 mg beta-1b every other day.

Another particular embodiment of the invention is related to a method for treating multiple sclerosis, in a patient in need thereof, comprising administering to the patient about 14 mg teriflunomide once a day and about 30 mcg beta-1a once a week.

Another particular embodiment of the invention is related to a method for treating multiple sclerosis, in a patient in need thereof, comprising administering to the patient about 14 mg teriflunomide once a day and about 22 mcg beta-1a three times a week.

Another particular embodiment of the invention is related to a method for treating multiple sclerosis, in a patient in need thereof, comprising administering to the patient about 14 mg teriflunomide once a day and about 44 mcg beta-1a three times a week.

Another particular embodiment of the invention is related to a method for treating multiple sclerosis, in a patient in need thereof, comprising administering to the patient about 14 mg teriflunomide once a day and about 0.25 mg beta-1b every other day.

Another particular embodiment of the invention is related to a method for treating multiple sclerosis, wherein the teriflunomide or the pharmaceutically acceptable salt is administered orally.

Another particular embodiment of the invention is related to a method for treating multiple sclerosis, wherein the interferon beta-1a is administered intramuscularly or subcutaneously.

Another particular embodiment of the invention is related to a method for treating multiple sclerosis, wherein the interferon beta-1b is administered subcutaneously.

Another particular embodiment of the invention is related to a method for reducing the number of T1-Gd lesions in a patient afflicted with multiple sclerosis, comprising administering to the patient about 7 mg or 14 mg teriflunomide or a pharmaceutically acceptable salt thereof, and a pharmaceutically effective amount of interferon beta-1a or 1b.

Another particular embodiment of the invention is related to a method for reducing the number of T1-Gd lesions in a patient afflicted with multiple sclerosis, comprising administering to the patient about 7 mg or 14 mg teriflunomide once a day, and a stable dose of interferon beta-1a or 1b.

Another particular embodiment of the invention is related to a clinically proven safe and effective method for reducing the number of T1-Gd lesions in a patient afflicted with multiple sclerosis, comprising administering to the patient about 7 mg or 14 mg teriflunomide once a day, and a stable dose of interferon beta-1a or 1b.

Another particular embodiment of the invention is related to a method for reducing the number of T1-Gd lesions in a patient afflicted with multiple sclerosis, comprising administering to the patient about 7 mg or 14 mg teriflunomide or a pharmaceutically acceptable salt thereof, and a pharmaceutically effective amount of interferon beta-1a or 1b, wherein the method reduces more T1-Gd lesions in the patient than when the patient is administered a stable dose of interferon beta-1a or 1b alone.

Another particular embodiment of the invention is related to a method for reducing the number of T1-Gd lesions in a patient afflicted with multiple sclerosis, comprising administering to the patient about 7 mg or 14 mg teriflunomide once a day, and a stable dose of interferon beta-1a or 1b, wherein the method reduces more T1-Gd lesions in the patient than when the patient is administered a stable dose of interferon beta-1a or 1b alone.

Another particular embodiment of the invention is related to a clinically proven safe and effective method for reducing the number of T1-Gd lesions in a patient afflicted with multiple sclerosis, comprising administering to the patient about 7 mg or 14 mg teriflunomide once a day, and a stable dose of interferon beta-1a or 1b, wherein the method reduces more T1-Gd lesions in the patient than when the patient is administered a stable dose of interferon beta-1a or 1b alone.

Another particular embodiment of the invention is related to a method for reducing the number of T1-Gd lesions in a patient afflicted with multiple sclerosis, comprising administering to the patient about 7 mg teriflunomide once a day and about 30 mcg beta-1a once a week.

Another particular embodiment of the invention is related to a method for reducing the number of T1-Gd lesions in a patient afflicted with multiple sclerosis, comprising administering to the patient about 7 mg teriflunomide once a day and about 22 mcg beta-1a three times a week.

Another particular embodiment of the invention is related to a method for reducing the number of T1-Gd lesions in a patient afflicted with multiple sclerosis, comprising administering to the patient about 7 mg teriflunomide once a day and about 44 mcg beta-1a three times a week.

Another particular embodiment of the invention is related to a method for reducing the number of T1-Gd lesions in a patient afflicted with multiple sclerosis, comprising administering to the patient about 7 mg teriflunomide once a day and about 0.25 mg beta-1b every other day.

Another particular embodiment of the invention is related to a method for reducing the number of T1-Gd lesions in a patient afflicted with multiple sclerosis, comprising administering to the patient about 14 mg teriflunomide once a day and about 30 mcg beta-1a once a week.

Another particular embodiment of the invention is related to a method for reducing the number of T1-Gd lesions in a patient afflicted with multiple sclerosis, comprising administering to the patient about 14 mg teriflunomide once a day and about 22 mcg beta-1a three times a week.

Another particular embodiment of the invention is related to a method for reducing the number of T1-Gd lesions in a patient afflicted with multiple sclerosis, comprising administering to the patient about 14 mg teriflunomide once a day and about 44 mcg beta-1a three times a week.

Another particular embodiment of the invention is related to a method for reducing the number of T1-Gd lesions in a patient afflicted with multiple sclerosis, comprising administering to the patient about 14 mg teriflunomide once a day and about 0.25 mg beta-1b every other day.

Another particular embodiment of the invention is related to a method for reducing the number of T1-Gd lesions in a patient afflicted with multiple sclerosis, wherein the teriflunomide or the pharmaceutically acceptable salt is administered orally.

Another particular embodiment of the invention is related to reducing the number of T1-Gd lesions in a patient afflicted with multiple sclerosis, wherein the interferon beta-1a is administered intramuscularly or subcutaneously.

Another particular embodiment of the invention is related to a method for reducing the number of T1-Gd lesions in a patient afflicted with multiple sclerosis, wherein the interferon beta-1b is administered subcutaneously.

It is found that the addition of teriflunomide to stable-dosed interferon improves disease control beyond interferon alone, in terms of MRI activity, without added concerns regarding safety and tolerability.

### Example

A placebo-controlled, double-blinded, randomized, 3-parallel group stratified study was conducted in relapsing-remitting multiple sclerosis patient who were concurrently on a stable dose of interferon beta. The dose level of interferon beta was defined as follows:
- Low dose: AVONEX® (interferon beta-1a) 30 mcg once a week intramuscularly, or REBIF® (interferon beta-1a) 22 mcg three times per week subcutaneously.
- High dose: REBIF® 44 mcg three times per week subcutaneously, or BETASERON® (interferon beta-1b) 0.25 mg every other day subcutaneously.

Around 40 patients were treated in each treatment group (placebo: 41, 7 mg: 37, and 14 mg: 38). A third of patients in each group were treated with low dose interferon beta 1a. The demographic and disease baseline characteristics were generally comparable amount the 3 treatment groups. The mean age of the study population was 40.1 years. The majority of patients were female (69.8%) and had a relapsing remitting type of MS (87.9%), with a diagnosis of MS since around 8 years ago and around 40% of patients had no relapse in the previous year. The base line Expanded Disability Status Scale (EDSS) score was similar between treatment groups (around 2.5).

Method:
Safety was evaluated from Treatment Emergent Adverse Event (TEAE), physical examination and laboratory data (e.g. Liver Function Test, every 2-8 weeks), ECG and abdominal ultrasound (24-weeks). Brain MRI (magnetic resonance image) activity, including T1-gadolinium (T1-Gd) with central reading, as well as relapses and EDSS were recorded every 8-week.

Results:
Safety: Approximately 90% of patients completed the 24-week treatment period in each group. Treatment was prematurely discontinued for TEAE in one patient in each group. The proportion of patients with TEAE due to increased ALT/SGPT (Alanine Transaminase/Serum Glutamic Pyruvic Transaminase) was higher on 14mg (28.9%) than on 7 mg (13.5%) or placebo (12.2%). Among them, the proportion of patients with ALT/SGPT greater than 3xULN (three times above the Upper Limit of Normal range for the central laboratory) was low (4.8% in placebo; 0% in 7 mg; 5.2% in 14 mg) with one treatment discontinuation from placebo and one from 14mg. There was no case of concomitant bilirubin increase. The proportion of patients with TEAE potentially related to immunosuppression (including white blood cell counts, infections and infestations) was slightly higher in the teriflunomide groups (placebo: 32%, 7 mg: 49%, 14 mg: 47%). Among these events upper respiratory tract infections (nasopharyngitis, sinusitis) appeared to be more frequent at 14 mg (23.7%) than PBO (14.6%) and 7mg (10.8%). None of these events led to treatment discontinuation.
Efficacy: The number of T1-Gd lesions were significantly reduced in both teriflunomide arms, relative to placebo (7 mg: 56% and 14 mg: 81%; all p<0.001). The proportion of patients free of T1-Gd lesions was higher in both teriflunomide arms (placebo: 58%, 7 mg: 70%, 14 mg: 82%). A few relapses were reported during the 24-week period (5 on placebo, 5 on 7mg and 2 on 14mg).
Conclusion: The addition of teriflunomide to stable-dosed interferon beta significantly improved disease control beyond interferon beta alone, as evaluated by T1-gadolinium MRI activity, with acceptable safety and tolerability over 24 weeks of treatment.

## Claims

1. A method for treating multiple sclerosis, in a patient in need thereof, comprising administering to the patient about 7 mg or about 14 mg teriflunomide, and a pharmaceutically effective amount of interferon beta-1a or 1b.

2. The method according to claim 1, comprising administering to the patient about 7 mg or about 14 mg teriflunomide once a day, and a stable dose of interferon beta-1a or 1b.

3. A clinically proven safe and effective method for treating multiple sclerosis, in a patient in need thereof, comprising administering to the patient about 7 mg or 14 mg teriflunomide once a day, and a stable dose of interferon beta-1a or 1b.

4. A method for reducing the number of T1-Gd lesions in a patient afflicted with multiple sclerosis comprising administering to the patient about 7 mg or 14 mg teriflunomide or a pharmaceutically acceptable salt thereof, and a pharmaceutically effective amount of interferon beta-1a or 1b.

5. The method according to claim 4, comprising administering to the patient about 7 mg or 14 mg teriflunomide once a day, and a stable dose of interferon beta-1a or 1b.

6. A clinically proven safe and effective method for reducing the number of T1-Gd lesions in a patient afflicted with multiple sclerosis comprising administering to the patient about 7 mg or 14 mg teriflunomide once a day, and a stable dose of interferon beta-1a or 1b.

7. A method for reducing the number of T1-Gd lesions in a patient afflicted with multiple sclerosis, comprising administering to the patient about 7 mg or 14 mg teriflunomide or a pharmaceutically acceptable salt thereof, and a pharmaceutically effective amount of interferon beta-1a or 1b, wherein the method reduces more T1-Gd lesions in the patient than when the patient is administered a stable dose of interferon beta-1a or 1b alone.

8. The method according to claim 7, comprising administering to the patient about 7 mg or 14 mg teriflunomide once a day, and a stable dose of interferon beta-1a or 1b, wherein the method reduces more T1-Gd lesions in the patient than when the patient is administered a stable dose of interferon beta-1a or 1b alone.

9. A clinically proven safe and effective method for reducing the number of T1-Gd lesions in a patient afflicted with multiple sclerosis, comprising administering to the patient about 7 mg or 14 mg teriflunomide once a day, and a stable dose of interferon beta-1a or 1b, wherein the method reduces more T1-Gd lesions in the patient than when the patient is administered a stable dose of interferon beta-1a or 1b alone.

10. The method according to claims 1-9, comprising administering to the patient about 7 mg teriflunomide once a day and about 30 mcg beta-1a once a week.

11. The method according to claims 1-9, comprising administering to the patient about 7 mg teriflunomide once a day and about 22 mcg beta-1a three times a week.

12. The method according to claims 1-9, comprising administering to the patient about 7 mg teriflunomide once a day and about 44 mcg beta-1a three times a week.

13. The method according to claims 1-9, comprising administering to the patient about 7 mg teriflunomide once a day and about 0.25 mg beta-1b every other day.

14. The method according to claims 1-9, comprising administering to the patient about 14 mg teriflunomide once a day and about 30 mcg beta-1a once a week.

15. The method according to claims 1-9, comprising administering to the patient about 14 mg teriflunomide once a day and about 22 mcg beta-1a three times a week.

16. The method according to claims 1-9, comprising administering to the patient about 14 mg teriflunomide once a day and about 44 mcg beta-1a three times a week.

17. The method according to claims 1-9, comprising administering to the patient about 14 mg teriflunomide once a day and about 0.25 mg beta-1b every other day.

18. The method according to claims 1-9, wherein the teriflunomide or the pharmaceutically acceptable salt is administered orally.

19. The method according to claims 1-9, wherein the interferon beta-1a is administered intramuscularly or subcutaneously.

20. The method according to claims 1-9, wherein the interferon beta-1b is administered subcutaneously.
